# EUROPEAN PATENT APPLICATION

(11) **EP 4 745 889 A1**
(43) Date of publication of application: **20.05.2026**
(21) Application number: 25214020.7
(22) Date of filing: 06.11.2025
(51) Int. Cl.: G06T 7/55, A61C 9/00

(54) **METHOD AND APPARATUS FOR PROCESSING INTRAORAL SCAN IMAGE**

(30) Priority: 19.11.2024 KR 20240164936
(71) Applicant: Huvitz Co., Ltd., Anyang-si, Gyeonggi-do 14055 (KR)
(72) Inventor: IM, Jong Hyeock, 14055 Anyang-si (KR); CHO, Min Soo, 14055 Anyang-si (KR); LEE, Weon Joon, 14055 Anyang-si (KR)
(74) Representative: Henkel & Partner mbB

(57) **Abstract**

A method and apparatus for processing intraoral scan images capable of improving visibility of insufficiently scanned areas are disclosed. The method for processing intraoral scan image includes the steps of: positioning an intraoral scanning unit (50) inside an oral cavity and scanning an oral structure to obtain a primary shape image (30) of the oral structure including one or more insufficiently scanned areas (32); selecting one insufficiently scanned area (32a) from among the one or more insufficiently scanned areas (32); generating a secondary shape image (40) by moving the primary shape image (30) so as to increase an identifiability of the selected insufficiently scanned area (32a); setting a position of the insufficiently scanned area (32a) from the secondary shape image (40); when the position of the insufficiently scanned area (32a) is set, additionally scanning the selected insufficiently scanned area (32a) to obtain an additional scan shape image of the insufficiently scanned area (32a); and integrating the obtained additional scan shape image of the insufficiently scanned area (32a) with the primary shape image (30) of the oral structure to obtain a tertiary shape image (42) in which an image of the insufficiently scanned area (32a) is supplemented.

## Description

### CROSS-REFERENCE TO RELATED APPLICATION

This application claims benefit of priority to Korean Patent Application No. 10-2024-0164936 filed on November 19, 2024, the entire contents of which are incorporated herein by reference.

### FIELD OF THE DISCLOSURE

The present invention relates to processing intraoral scan images, and more particularly, to a method and apparatus for processing intraoral scan images capable of improving visibility of insufficiently scanned area.

### BACKGROUND

In general, dental clinics examine the shape of teeth or gums in a patient's oral cavity and diagnose the patient's oral condition or fabricate prosthetics based thereon. To obtain the shape of teeth and gums in the oral cavity, optical intraoral scanners that optically scan and capture the interior shape image of the patient's oral cavity without physical contact to detect the shape of oral structures have recently been used.

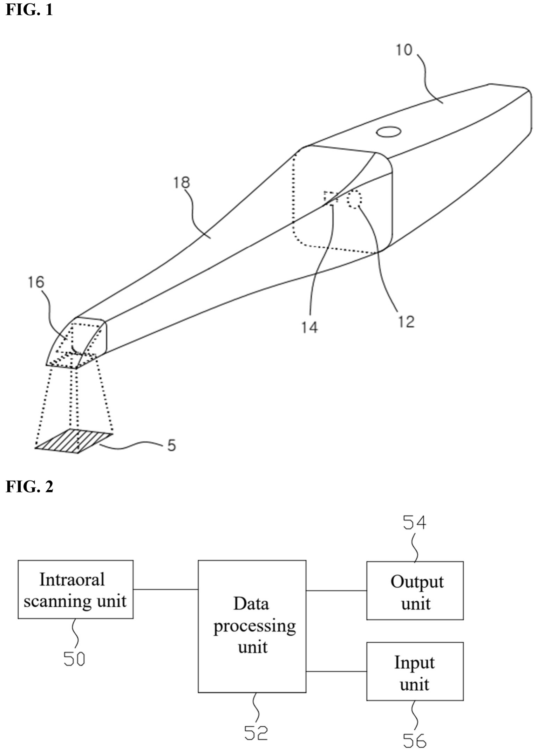

FIG. 1 is a perspective view of the appearance of a conventional optical intraoral scanner for dental use. As shown in FIG. 1, a conventional intraoral scanner includes a measurement light source (12) for irradiating measurement light onto an oral structure, an image detector (14) for detecting reflected light formed by the reflection of the measurement light from the oral structure, a scanner body (10) accommodating the measurement light source (12) and the image detector (14), and an intraoral tip (18) equipped with a reflection mirror (16) that reflects the measurement light generated from the measurement light source (12) to irradiate the oral structure and reflects the reflected light from the oral structure to guide it to the image detector (14).

The intraoral tip (18) of the intraoral scanner is positioned inside the patient's oral cavity, measurement light is irradiated onto a predetermined region (5) inside the oral cavity using the measurement light source (12), and then the reflected light generated by the reflection of the measurement light from the region (5) is detected using the image detector (14). At this time, since the shape of the reflected light changes according to the shape of the oral structure, the three-dimensional shape of the oral structure can be detected by analyzing the reflected light.

As shown in FIG. 1, since the measurement light irradiated into the oral cavity through the intraoral tip (18) is small in size, only a partial region (5) of the oral structure can be detected. Therefore, three-dimensional shape image data, i.e., a patch, for the partial region (5) of the oral structure is obtained, then an adjacent region is scanned to obtain a patch for the adjacent region, and the obtained patches are connected to obtain a three-dimensional shape image of the entire oral structure.

In the case of such dental intraoral scanners, since it is difficult to uniformly scan the narrow and complex structure of the oral cavity without omission through a limited number of scans, insufficiently scanned areas are likely to occur in the obtained three-dimensional shape image. Therefore, the user reviews the three-dimensional shape image of the entire oral structure obtained from the intraoral scanner, identifies the insufficiently scanned areas, rescans the corresponding insufficiently scanned areas to additionally obtain three-dimensional shape image data of the insufficiently scanned areas, and uses this to supplement the three-dimensional shape image of the oral structure.

However, in general, since insufficiently scanned areas exist in several locations, there is a problem in that it is difficult to accurately identify the positions of the several insufficiently scanned areas from the three-dimensional shape image of a conventional oral structure.

### [Prior Art Documents]

(Patent Document 1) Korean Patent No. 10-1874547
(Patent Document 2) Korean Patent No. 10-2033426

### SUMMARY OF THE DISCLOSURE

### TECHNICAL OBJECTS

An object of the present invention is to provide a method and apparatus for processing intraoral scan images that can easily identify the positions of insufficiently scanned areas when obtaining a three-dimensional shape image of an oral structure using an intraoral scanner, and improve the quality of the intraoral scan images.

### TECHNICAL SOLUTION

To achieve the above object, the present invention provides a method for processing intraoral scan images, comprising the steps of : positioning an intraoral scanning unit (50) inside an oral cavity and scanning an oral structure to obtain a primary shape image (30) of the oral structure including one or more insufficiently scanned areas (32); selecting one insufficiently scanned area (32a) from among the one or more insufficiently scanned areas (32); generating a secondary shape image (40) by moving the primary shape image (30) so as to increase an identifiability of the selected insufficiently scanned area (32a); setting a position of the insufficiently scanned area (32a) from the secondary shape image (40); when the position of the insufficiently scanned area (32a) is set, additionally scanning the selected insufficiently scanned area (32a) to obtain an additional scan shape image of the insufficiently scanned area (32a); and integrating the obtained additional scan shape image of the insufficiently scanned area (32a) with the primary shape image (30) of the oral structure to obtain a tertiary shape image (42) in which an image of the insufficiently scanned area (32a) is supplemented.

The present invention also provides an apparatus for processing intraoral scan images, comprising: an intraoral scanning unit (50) configured to optically scan an oral structure to obtain a primary shape image (30) of the oral structure including insufficiently scanned areas (32); a data processing unit (52) configured to (i) generate a secondary shape image (40) by moving the primary shape image (30) so as to increase an identifiability of an insufficiently scanned area (32a) selected by a user, and (ii) integrate an additional scan shape image of the selected insufficiently scanned area (32a) with the primary shape image (30) to generate a tertiary shape image (42) in which an image of the insufficiently scanned area (32a) is supplemented; an output unit (54) configured to display the shape images (30, 40, 42) of the oral structure obtained from the data processing unit (52) on a screen; and an input unit (56) configured to input user commands including selection of the insufficiently scanned area (32a).

### EFFECTS OF THE DISCLOSURE

According to the method and apparatus for processing intraoral scan images of the present invention, when obtaining a three-dimensional shape image of an oral structure using an intraoral scanner, the positions of insufficiently scanned areas can be easily identified, and the quality of the intraoral scan images can be improved.

### BRIEF DESCRIPTION OF THE DRAWINGS

FIG. 1 is a perspective view of the appearance of a conventional optical intraoral scanner for dental use.
FIG. 2 is a diagram showing the configuration of an apparatus for processing intraoral scan images according to an embodiment of the present invention.
FIGS. 3 and 4 are a flowchart and diagrams, respectively, for explaining a method for processing intraoral scan images according to an embodiment of the present invention.

### DETAILED DESCRIPTION OF THE PREFERRED EMBODIMENTS

Hereinafter, the present invention will be described in detail with reference to the accompanying drawings.

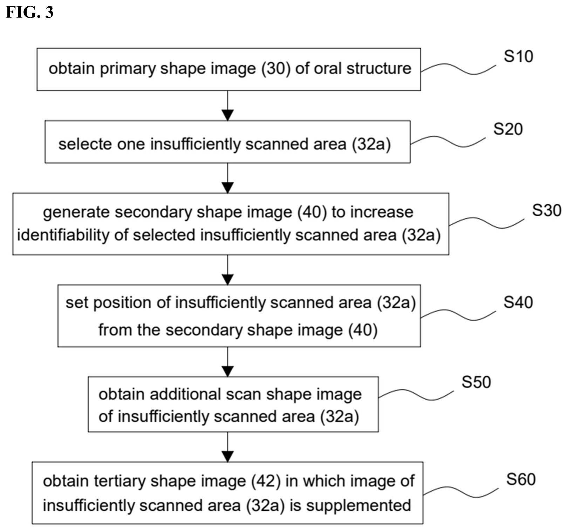

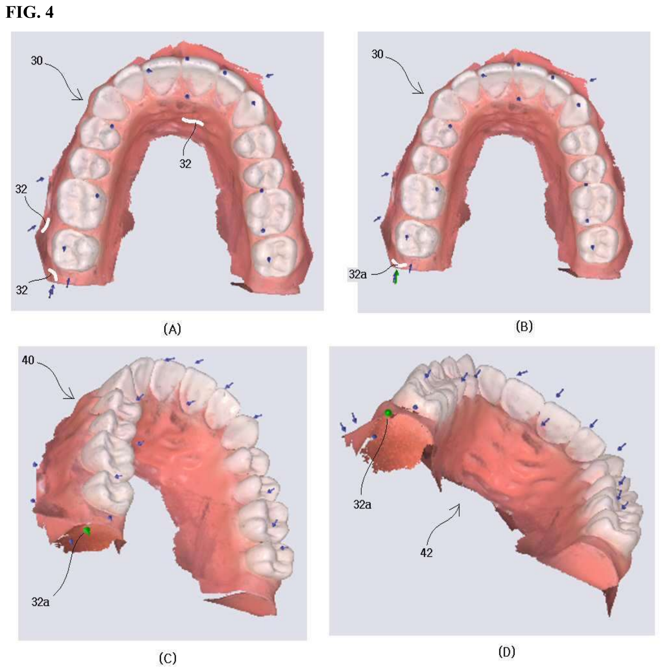

FIG. 2 is a diagram showing the configuration of an apparatus for processing intraoral scan images according to an embodiment of the present invention, and FIGS. 3 and 4 are a flowchart and diagrams, respectively, for explaining a method for processing intraoral scan images according to an embodiment of the present invention. The apparatus for processing intraoral scan images according to the present invention is a device for obtaining a three-dimensional shape image of a patient's oral cavity to assist in dental treatments such as orthodontic treatment, prosthetic fabrication, and denture fabrication in dentistry.

As shown in FIG. 2, the apparatus for processing intraoral scan images according to the present invention includes an intraoral scanning unit (50), a data processing unit (52), an output unit (54), and an input unit (56).

Referring to FIGS. 2 and 4, the intraoral scanning unit (50) optically scans the oral structure, for example, teeth, gums, maxilla, mandible, etc., to obtain a primary shape image (30, see A in FIG. 4) of the oral structure including one or more insufficiently scanned areas (32). The intraoral scanning unit (50) may be, for example, a conventional three-dimensional (3D) intraoral scanner or 3D model scanner as shown in FIG. 1.

The data processing unit (52) processes the three-dimensional shape image of the oral structure obtained from the intraoral scanning unit (50), specifically, improves or supplements the image of the insufficiently scanned areas (32), to generate an improved three-dimensional shape image (42, see D in FIG. 4) of the oral structure necessary for the patient's examination or treatment. The data processing unit (52) may be a computer processor capable of performing processing (i.e., identification, movement, comparison, matching, etc.) of the shape image data.

Specifically, the data processing unit (52) generates a secondary shape image (40) by moving the primary shape image (30) so as to increase the identifiability of the insufficiently scanned area (32a) selected by the user, and integrates an additional scan shape image of the selected insufficiently scanned area (32a) with the primary shape image (30) to generate a tertiary shape image (42) in which the image of the insufficiently scanned area (32a) is supplemented.

The output unit (54) is a device that displays on a screen the shape images (30, 40, 42) of the oral structure obtained from the data processing unit (52), i.e., the processing process of the intraoral scan images, the shape images of the oral structure obtained thereby, etc. The output unit (54) is, for example, a conventional screen display device such as a liquid crystal display (LCD) device.

The input unit (56) is a device for inputting user commands including selection of the insufficiently scanned area (32a). The input unit (56) is, for example, a device for inputting user operation commands such as movement, rotation, and selection of insufficiently scanned areas of the shape image of the oral structure on a user interface displayed on the screen of the output unit (54). As the input unit (56), for example, conventional input devices such as a keyboard or a mouse may be used.

A method for processing intraoral scan images according to an embodiment of the present invention will be described in detail with reference to FIGS. 3 and 4. To process intraoral scan images according to the present invention, first, the intraoral scanning unit (50) is positioned inside the oral cavity, and the oral structure is scanned to obtain a primary shape image (30) of the oral structure including one or more insufficiently scanned areas (32) (S10, see A in FIG. 4). For example, the primary shape image (30) is obtained by processing the shape image data of the oral structure obtained from the intraoral scanning unit (50) in the data processing unit (52) and displaying it on the output unit (54) (see A in FIG. 4).

Here, the primary shape image (30) refers to an unprocessed three-dimensional shape image (30) including one or more insufficiently scanned areas (32) obtained by the primary, i.e., initial, scan of the intraoral scanning unit (50). In the primary shape image (30), the insufficiently scanned areas (32) refer to areas where, due to insufficient scan data in the corresponding areas, a natural image of the oral structure, i.e., teeth or gums, etc., is not formed, and due to insufficient or absent data (i.e., images), unnatural images, such as white spaces, are displayed.

Here, the insufficiently scanned areas (32) may be identified from the primary shape image (30) visually based on the user's experience, or the insufficiently scanned areas (32) may be identified from the primary shape image (30) by a software program of the data processing unit (52) and displayed on the output unit (54). In the example shown in A of FIG. 4, the insufficiently scanned areas (32) identified by the software program of the data processing unit (52) are indicated by blue arrows. In this case, the user can recognize that insufficiently scanned areas (32) exist in the portions indicated by the arrows in A of FIG. 4.

Next, to additionally scan the insufficiently scanned areas, one insufficiently scanned area (32a) is selected by the user from among the one or more insufficiently scanned areas (32) (S20, see B in FIG. 4). In B of FIG. 4, the selected insufficiently scanned area (32a) is indicated by a green arrow.

When one insufficiently scanned area (32a) is selected in this way, a secondary shape image (40) is generated by moving the primary shape image (30) so as to increase the identifiability of the selected insufficiently scanned area (32a) (S30, see C in FIG. 4).

Here, "movement" includes linear movement and rotational movement. For example, when the selected insufficiently scanned area (32a) is on a side of the primary shape image (30) of the oral structure (see B in FIG. 4), by rotating the primary shape image (30) to generate a secondary shape image (40) in which the selected insufficiently scanned area (32a) is displayed relatively frontally (see C in FIG. 4), the selected insufficiently scanned area (32a) can be better identified. The movement of the primary shape image (30) changes the direction and position of the primary shape image (30) displayed on the output unit (54), i.e., rotates and/or moves the primary shape image (30), so that the selected insufficiently scanned area (32a) and its surroundings can be better identified. The movement can be performed by user input and a software program of the data processing unit (52).

Additionally, if necessary, along with the movement of the primary shape image (30), the color of the selected insufficiently scanned area (32a) displayed in the secondary shape image (40) may be changed, or additional identification symbols may be added to further increase the identifiability of the selected insufficiently scanned area (32a).

After the secondary shape image (40) with increased identifiability of the selected insufficiently scanned area (32a) is generated in this way, the position of the insufficiently scanned area (32a) is set (i.e., determined) from the secondary shape image (40) (S40, see C in FIG. 4). In C of FIG. 4, the position of the insufficiently scanned area (32a) displayed in the secondary shape image (40) is indicated by a green dot. That is, the user can more accurately confirm or set the position of the insufficiently scanned area (32a) from the secondary shape image (40) having increased identifiability of the insufficiently scanned area (32a).

When the position of the insufficiently scanned area (32a) is set in this way, with reference thereto, the selected insufficiently scanned area (32a) is additionally scanned by the intraoral scanning unit (50) to obtain an additional scan shape image of the insufficiently scanned area (32a) (S50).

Finally, the obtained additional scan shape image of the insufficiently scanned area (32a) is integrated with the primary shape image (30) of the oral structure to obtain a tertiary shape image (42) in which the image of the insufficiently scanned area (32a) is supplemented, i.e., the visibility of the insufficiently scanned area (32a) is increased (S60, see D in FIG. 4).

According to the present invention, by moving and displaying the shape image (30) of the oral structure so as to improve the identifiability of the insufficiently scanned area (32a) selected by the user, the position of the insufficiently scanned area can be easily identified, and a shape image (42) of the oral structure having increased visibility of the insufficiently scanned area (32a) can be obtained.

Although the present invention has been described with reference to the accompanying drawings and exemplary embodiments, the present invention is not limited to the contents shown in the drawings and the above-described embodiments. Reference numerals are indicated in the following claims for better understanding, but the scope of the following claims is not limited to the reference numerals and the contents shown in the drawings, and should be interpreted to encompass all modifications, equivalent configurations, and functions of the exemplary embodiments.

## Claims

1. A method for processing intraoral scan images, comprising the steps of :
positioning an intraoral scanning unit (50) inside an oral cavity and scanning an oral structure to obtain a primary shape image (30) of the oral structure including one or more insufficiently scanned areas (32);
selecting one insufficiently scanned area (32a) from among the one or more insufficiently scanned areas (32);
generating a secondary shape image (40) by moving the primary shape image (30) so as to increase an identifiability of the selected insufficiently scanned area (32a);
setting a position of the insufficiently scanned area (32a) from the secondary shape image (40);
when the position of the insufficiently scanned area (32a) is set, additionally scanning the selected insufficiently scanned area (32a) to obtain an additional scan shape image of the insufficiently scanned area (32a); and
integrating the obtained additional scan shape image of the insufficiently scanned area (32a) with the primary shape image (30) of the oral structure to obtain a tertiary shape image (42) in which an image of the insufficiently scanned area (32a) is supplemented.

2. The method of claim 1, wherein the insufficiently scanned areas (32) are identified from the primary shape image (30) visually based on a user's experience.

3. The method of claim 1, wherein the insufficiently scanned areas (32) are identified from the primary shape image (30) by a software program of a data processing unit (52).

4. An apparatus for processing intraoral scan images, comprising:
an intraoral scanning unit (50) configured to optically scan an oral structure to obtain a primary shape image (30) of the oral structure including insufficiently scanned areas (32);
a data processing unit (52) configured to (i) generate a secondary shape image (40) by moving the primary shape image (30) so as to increase an identifiability of an insufficiently scanned area (32a) selected by a user, and (ii) integrate an additional scan shape image of the selected insufficiently scanned area (32a) with the primary shape image (30) to generate a tertiary shape image (42) in which an image of the insufficiently scanned area (32a) is supplemented;
an output unit (54) configured to display the shape images (30, 40, 42) of the oral structure obtained from the data processing unit (52) on a screen; and
an input unit (56) configured to input user commands including selection of the insufficiently scanned area (32a).

5. The apparatus of claim 4, wherein the data processing unit (52) is a computer processor capable of performing processing of shape image data.
